Europäisches Patentamt

European Patent Office

Office européen des brevets

(11) Numéro de publication: **0 234 143 B1**

(12) **FASCICULE DE BREVET EUROPEEN**

(45) Date de publication de fascicule du brevet:
21.08.91

(21) Numéro de dépôt: 86402691.9

(22) Date de dépôt: 04.12.86

(51) Int. Cl.⁵: **A61K 9/14**, A61K 35/78,
A61K 7/48

(54) Formes galéniques à base de plantes fraîches et leurs procédés de préparation.

(30) Priorité: 23.12.85 FR 8519031

(43) Date de publication de la demande:
02.09.87 Bulletin 87/36

(45) Mention de la délivrance du brevet:
21.08.91 Bulletin 91/34

(84) Etats contractants désignés:
BE CH DE ES GB IT LI LU

(56) Documents cités:
DE-A- 2 539 823
FR-A- 2 556 215
LU-A- 83 173

O.A. NEUMÜLLER: "Römpps Chemielexikon",
7ième édition, vol. 2, 1973, Franckh'sche Verlagshandlung, Stuttgart, DE

(73) Titulaire: **LABORATOIRE ARDEVAL**
**La Sigalière**
**F-07110 LARGENTIERE(FR)**

(72) Inventeur: **Lombard, André**
**14 Square Adanson**
**F-75005 Paris(FR)**

(74) Mandataire: **Orès, Bernard et al**
**Cabinet ORES 6, Avenue de Messine**
**F-75008 Paris(FR)**

## Description

La présente invention est relative à de nouvelles formes galéniques à base de plantes fraîches et à leurs procédés de préparation.

Depuis des temps immémoriaux, l'homme s'est servi de la plante pour soulager ses maux, pour se guérir, pour se stimuler. Actuellement encore les médicaments à base de plantes sont nombreux. Ils sont constitués soit de plantes séchées, soit de plantes fraîches broyées en suspension dans l'alcool ou autre solvant, soit des plantes macérées puis filtrées, soit des extraits de plantes obtenus par distillation ou par la concentration, soit encore des extraits obtenus par l'expression et la filtration. Toutefois aucune de ces formules n'est entièrement satisfaisante. En effet, la plante fraîche qui constitue un extraordinaire réservoir en différents principes actifs subit des transformations indésirables et parfois même toxiques aussi bien pendant les opérations de séchage et/ou d'extraction que pendant le stockage et lors de la conservation des différentes préparations obtenues (suspensions, teintures, huiles essentielles, extraits concentrés, etc...). En ce qui concerne la plante faîche il n'existe pas actuellement de forme stable. L'usage de la plante fraîche n'est actuellement rencontré que sous forme extemporanée que ce soit lors d'absorption par voie interne ou en application sous forme de cataplasmes par voie externe.

Toutes les formes galéniques d'un usage commode et facile telles que crèmes, pâtes et pommades ne contiennent que des extraits de plantes : extraits fluides, secs, aqueux, alcooliques, hydroalcooliques glycériques, etc... Toutes ces formes ne contiennent que les parties de la plante stables au séchage, à la concentration ou à l'extraction, ou bien les parties de la plante extractibles par les différents solvants choisis.

La présente invention s'est par conséquent fixé pour but de pourvoir à une nouvelle forme galénique d'un usage facile et commode qui répond mieux aux nécessités de la pratique que les formes antérieurement connues, notamment en ce qu'elles contiennent toutes les parties de la plante fraîche, c'est-à-dire la totalité des principes actifs contenus dans la plante.

La présente invention a pour objet une forme galénique à base de plante(s) fraîche(s), caractérisée en ce qu'elle se présente sous forme pâteuse et dérive d'un cryobroyat de ladite plante obtenu par congélation brutale de la plante fraîchement récoltée, à une température comprise entre -80° C et -120° C, suivi par au moins un broyage cryogénique de ladite plante congelée puis d'un mélange de 5 à 75 % en poids dudit cryobroyat de plante avec 1 à 5 % en poids d'un gélifiant dans un mélangeur sous gaz inerte à une température comprise entre -5 et -30° C et d'une remise à température ambiante, pour être ainsi stabilisée.

Conformément à l'invention, le gélifiant est pris dans le groupe qui contient les galactomannanes, les farines naturelles, la cellulose et ses dérivés, les acides polyacryliques réticulés, les silices, la gélatine, l'agar agar, les gommes adragantes ou arabiques, les alginates, les pectines, les palmitostéarates, les oléates, les laurates et les myristates.

Selon un mode de réalisation avantageux de l'objet de l'invention, la plante fraîche congelée puis cryobroyée avant son incorporation dans la forme pâteuse est préalablement micronisée.

Conformément à l'invention les nouvelles formes galéniques contiennent en outre des conservateurs (0,15 à 0,5 % en poids).

Les nouvelles formes galéniques peuvent contenir en outre :

dans le cas d'un usage interne 0,1 à 10 % en poids d'un édulcorant et des aromatisants (0,13 à 2 % en poids),

et dans le cas d'un usage externe 5 à 30 % en poids de glycérol.

La présente invention est également relative à un procédé de préparation de la forme galénique, caractérisé en ce que :

- la plante fraîchement récoltée est fragilisée par congélation brutale à une température comprise entre -80° C et -120° C ;
- ladite plante congelée est soumise à au moins un broyage cryogénique jusqu'à l'obtention d'une granulométrie de particules de l'ordre de 100 $\mu$m ;
- en ce qu'on mélange 5 à 75 % en poids de cryobroyat de plantes avec 1 à 5 % en poids d'un gélifiant dans un mélangeur sous gaz inerte à une température comprise entre -5° C et -30° C ; et
- en ce qu'on remet ledit cryobroyat à température ambiante, pour sa stabilisation.

Selon un mode de réalisation avantageux du procédé objet de la présente invention, la plante à traiter - notamment dans le cas de racines - est prédécoupée avant la cryocongélation de manière que cette dernière soit rapide, même au coeur de la matière.

La nouvelle forme galénique qui préserve l'intégralité des principes actifs de la plante fraîche peut être utilisée avec succès, aussi bien en pharmacie qu'en cosmétologie et en diététique.

D'une manière générale, pour des préparations pharmaceutiques, la posologie pour une forme contenant environ 50 % de plante fraîche peut être de 1 à 2 cuillères à café par jour.

Outre les dispositions qui précèdent, l'invention comprend encore d'autres dispositions qui ressortiront de la description qui va suivre.

L'invention pourra être mieux comprise à l'aide u complément de description qui va suivre, qui se réfère à des exemples de formulation et d'utilisation aussi bien en pharmacie qu'en cosmétologie et en diététique.

## EXEMPLE 1

Racines fraîches de Bardane.

La racine de Bardane fraîche récoltée au printemps est prédécoupée moins de 24 heures après sa récolte puis fragilisée par congélation brutale à -100° par l'azote liquide. Après broyage dans un broyeur à vis, le cryobroyat est stabilisé lors du retour à température ambiante, puis incorporé dans une pâte qui comprend :

Racines de Bardane fraîches    15 %
Acide polyacrylique réticulé    2 %
Glycérol    18 %
Conservateur codex    0,19 %
Neutralisants    pH 6 ± 0,5
Eau q.s.p. pour    100 %

Indications et mode d'emploi.

Les applications locales de la pâte de Bardane sont un complément efficace au traitement général dans les affections de la peau, telles que :
- l'acné, la séborrhée du visage, l'eczéma sec.

On applique une mince couche de la pâte de Bardane sur les zones à traiter et on laisse en contact de 1/4 d'heure à 1/2 heure.

On rince légèrement à l'eau tiède, puis on renouvelle chaque jour les applications.

En ce qui concerne les abcès, les furoncles et les panaris et pour hâter la maturation, on applique une bonne couche de la pâte de Bardane, puis on recouvre d'une compresse légèrement imbibée d'eau chaude. On maintient en place en renouvelant le pansement fréquemment (6 à 8 fois par jour).

## EXEMPLE 2

Tiges stériles de Prêle des Champs

On opère d'une manière analogue à celle indiquée dans l'Exemple 1.

Indications et mode d'emploi.

Les propriétés antidégénératives, réminéralisantes et cicatrisantes de la Prêle des Champs sont utilisées en applications locales, en complément des traitements généraux de diverses affections, telles que :
- le vieillissement cutané, les rides du visage et du cou,

- les vergétures.

On applique sur les zones à traiter une mince couche de cataplasme de Prêle, on masse légèrement pour favoriser une bonne pénétration et on laisse en place au minimum 1/4 d'heure ; on rince éventuellement à l'eau tiède.

On renouvelle 1 ou 2 fois par jour en cures prolongées (1 à 2 mois).

En ce qui concerne les douleurs rhumatismales (arthrite, arthrose):

On applique une mince couche de cataplasme de Prêle sur les articulations douloureuses, on recouvre d'une compresse légèrement imbibée d'eau chaude et on laisse en place en maintenant avec une bande pendant 1/4 d'heure à 1 heure. On rince éventuellement à l'eau tiède.

On renouvelle l'application 1 à 3 fois par jour.

## EXEMPLE 3

Fucus (algues fraîches stabilisées)

On opère comme indiqué dans l'Exemple 1, avec cette différence que l'on utilise un broyeur à couteaux et on opère 2 passages successifs de manière à obtenir une granulométrie de particules de l'ordre de 100 μm.

Indications et mode d'emploi.

Les applications locales de cataplasme de Fucus sont un complément efficace des traitements généraux de l'obésité et de la cellulite.

On applique une mince couche de cataplasme sur les zones à affiner : cuisses, hanches, ventre, etc... on masse éventuellement doucement pour favoriser la pénétration. On laisse en contact au moins 1/4 d'heure et on rince éventuellement à l'eau tiède ou froide.

## EXEMPLE 4

Cataplasmes au Mélilot

On récolte la plante fleurie puis on procède comme indiqué précédemment.

Indications et mode d'emploi.

Les applications locales de cataplasme de Mélilot sont un complément efficace au traitement général des troubles vasculaires veinolymphatiques : action antioedemateuse et veinotonique.

On applique sur les jambes une mince couche de cataplasme de plantes fraîches, on maase légèrement de bas en haut et on laisse en place 1/4 d'heure ou plus.

On rince à l'eau tiède ou on douche les jambes à l'eau tiède plus froide.

On renouvelle les applications en moyenne 1 à 2 fois par jour.

## EXEMPLE 5

### Pâte aux Marrons d'Inde

Les graines du Marron d'Inde fraîchement récoltées (en général en Septembre - Octobre) sont traitées comme indiqué dans l'Exemple 3. On utilise pour la composition de la pâte de la gomme arabique comme gélifiant (1,8%).

### Indications et mode d'emploi

Les applications locales de la pâte de Marron d'Inde sont un complément efficace au traitement général de l'insuffisance veineuse : (jambes lourdes, varices).

On applique sur les jambes une mince couche de la pâte conformément à l'invention, on masse éventuellement légèrement et de bas en haut, on laisse en place 1/4 d'heure. On rince à l'eau tiède, ou mieux on douche les jambes à l'eau tiède puis froide.

On renouvelle les applications en moyenne 1 à 2 fois par jour.

En ce qui concerne les engelures :

On applique une couche de la pâte de Marron d'Inde, puis on recouvre d'une compresse légèrement humide et on laisse en place de 1/4 d'heure à 1/2 heure.

On rince à l'eau tiède.

On renouvelle les applications 2 fois par jour.

## EXEMPLE 6

### Cataplasme à l'Ulmaire

La plante est récoltée avant l'épanouissement complet des fleurs et on détache avec des ciseaux les petites cymes ou sommités terminales. On fragilise par congélation à -110° C puis on opère un cryobroyage dans un broyeur à marteaux à une température voisine de -50° C. La composition du cataplasme est par exemple la suivante :

Sommités fleuries fraîches stabilisées d'Ulmaire   15 %
Pectine de pomme   1,5 %
Glycérol   15 %
Conservateur codex   0,5 %
Eau q.s.p.   100 %

### Indications et mode d'emploi

Les applications locales de cataplasme d'Ulmaire sont un complément efficace du traitement général des affections suivantes : les douleurs rhumatismales, (arthrite, arthrose), les brulûres, les petites coupures, les irrita-tions de la peau.

On applique une mince couche de cataplasme d'Ulmaire sur la zone douloureuse, on recouvre d'une compresse de gaze imbibée d'eau chaude et on laisse en place en maintenant éventuellement avec une bande, pendant 1/4 d'heure à 1 heure.

On renouvelle l'application 1 à 3 fois par jour.

On rince éventuellement à l'eau tiède.

## EXEMPLE 7

### Gelée à l'Artichaut pour usage interne

Composition :

Artichaut cryobroyé   50 %
Agar - agar   5 %
Conservateur codex   0,2 %
Saccharine   4 %
Aromatisant (caramel)   1 %
Eau q.s.p.   100 %
Indications : Actions chlolalogue et cholérétique.
Posologie : 1 à 2 cuillères à café par jour.

## EXEMPLE 8
### Gelée de cynorrhodon frais

Les cynorrhodons frais récoltés encore rouges, luisants et lisses sont largement utilisés en diététique en tant que, par exemple, astringents actifs non désagréables.

Composition :
Cynorrhodon frais cryobroyé   60 %
Pectine de citron   3 %
Conservateur codex   0,2 %
Sucre   8 %
Aromes   0,5 %
Eau q.s.p.   100 %

Il résulte de la description qui précède que quels que soient les modes de mise en oeuvre, de réalisation et d'application adoptées, on obtient des pâtes, crèmes ou cataplasmes de conservation et d'application facile et qui contiennent intacts tous les principes actifs de la cellule fraîche vivante.

**Revendications**
**Revendications pour les Etats contractants suivants : BE, CH, DE, GB, IT, LI, LU**

1. Forme galénique à base de plante(s) fraîche(s), caractérisée en ce qu'elle se présente sous forme pâteuse et dérive d'un cryobroyat de ladite plante obtenu par congélation brutale de la plante fraîchement récoltée, à une température comprise entre -80°C et -120°C, suivi par au moins un broyage cryogénique de ladite plante congelée puis d'un mélange de 5 à 75 % en poids dudit cryobroyat de plante avec 1 à 5 % en poids d'un gélifiant dans un mélangeur sous gaz inerte à une température comprise entre -5 et -30°C et d'une remise à température ambiante, pour être ainsi stabilisée.

2. Forme galénique selon la revendication 1, caractérisée en ce que le gélifiant est pris dans le groupe qui contient les galactomannanes, les farines naturelles, la cellulose et ses dérivés, les acides polyacryliques réticulés, les silices, la gélatine, l'agaragar, les gommes adragantes ou arabiques, les alginates, les pectines, les palmitostéarates, les oléates, les laurates et les myristates.

3. Forme galénique selon la revendication 1, caractérisée en ce que la plante fraîche congelée puis cryobroyée est micronisée avant son incorporation dans la forme pâteuse.

4. Forme galénique selon l'une quelconque des revendications 1 à 3, caractérisée en ce qu'elle contient en outre des conservateurs (0,15 à 0,5 % en poids).

5. Forme galénique selon l'une quelconque des revendications 1 à 4, caractérisée en ce qu'elle contient dans le cas d'un usage interne 0,1 à 10 % en poids d'un édulcorant et des aromatisants (0,13 à 2 % en poids) et dans le cas d'un usage externe 5 à 30 % en poids de glycérol.

6. Forme galénique selon l'une quelconque des revendications 1 à 5, caractérisée en ce que le cryobroyat de plante présente une granulométrie de particules de l'ordre de 100 $\mu$m.

7. Utilisation de la forme galénique selon l'une quelconque des revendications 1 à 6 en application externe.

8. Utilisation de la forme galénique selon l'une quelconque des revendications 1 à 6 en application interne.

9. Utilisation de la forme galénique selon l'une quelconque des revendications 1 à 6 en pharmacie.

10. Utilisation de la forme galénique selon l'une quelconque des revendications 1 à 6 en cosmétologie.

11. Utilisation de la forme galénique selon l'une quelconque des revendications 1 à 6 en diététique.

12. Procédé de préparation de la forme galénique selon l'une quelconque des revendications 1 à 6, caractérisé en ce que :
    - la plante fraîchement récoltée est fragilisée par congélation brutale à une température comprise entre -80°C et -120°C ;
    - ladite plante congelée est soumise à au moins un broyage cryogénique jusqu'à l'obtention d'une granulométrie de particules de l'ordre de 100 $\mu$m ;
    - en ce qu'on mélange 5 à 75 % en poids de cryobroyat de plantes avec 1 à 5 % en poids d'un gélifiant dans un mélangeur sous gaz inerte à une température comprise entre -5°C et -30°C ; et
    - en ce qu'on remet ledit cryobroyat à température ambiante, pour sa stabilisation.

13. Procédé selon la revendication 12, caractérisé en ce que la plante à traiter - notamment dans le cas de racines - est prédécoupée avant la cryocongélation, de manière à ce que cette dernière soit rapide, même au coeur de la matière.

**Revendications pour l'Etat contractant suivant : ES**

1. Procédé de préparation de forme galénique à base de plantes fraîches se présentant sous forme pâteuse et contenant les plantes fraîchement récoltées, congelées puis cryobroyées, caractérisé en ce que la plante fraîchement récoltée est fragilisée par congélation brutale à une température comprise entre -80°C et -120°C, puis soumise à un broyage cryogénique, en ce que le cryobroyat est incorporé au gélifiant dans un mélangeur sous gaz inerte à une température comprise entre -5 et -30°C et en ce qu'il est ainsi stabilisé lors du retour à température ambiante.

2. Procédé selon la Revendication 1, caractérisé en ce que la plante à traiter - notamment dans le cas de racines - est prédécoupée avant la cryocongélation, de manière que cette dernière soit rapide, même au coeur de la matière.

3. Procédé selon l'une quelconque des Revendications 1 et 2, caractérisé en ce qu'un ou plusieurs passages dans le broyeur peuvent être réalisés, ceci afin d'obtenir une granulométrie de particules de l'ordre de 100 $\mu$m.

4. Procédé selon l'une quelconque des Revendications 1 à 3, caractérisé en ce que la forme pâteuse de plantes fraîches contient de 5 à 75 % en poids de Plantes et de 1 à 5 % en poids d'un gélifiant (naturel ou synthétique).

5. Procédé selon l'une quelconque des Revendications 1 à 4, caractérisé en ce que le gélifiant utilisé est pris dans le groupe qui contient les galactomannanes, les farines naturelles, la cellulose et ses dérivés, les acides polyacryliques réticulés, les silices, la gélatine, l'agaragar, les gommes adragantes ou arabiques, les alginates, les pectines, les palmitostéarates, les oléates, les laurates et les myristates.

6. Procédé selon l'une quelconque des Revendications 1 à 5, caractérisé en ce que la plante fraîche congelée puis cryobroyée avant son incorporation dans la forme pâteuse, est préalablement micronisée.

7. Procédé selon l'une quelconque des Revendications 1 à 6, caractérisé en ce que la forme pâteuse contenant les plantes, contient également des conservateurs (0,15 à 0,5 % en poids).

8. Procédé selon l'une quelconque des Revendications 1 à 7, caractérisé en ce que la forme pâteuse contenant les plantes, contient, dans le cas d'un usage interne 0,1 à 10 % en poids d'un édulcorant et des aromatisants (0,13 à 2 % en poids), et dans le cas d'un usage externe 5 à 30 % en poids de glycérol.

9. Application de formes pâteuses contenant des plantes selon l'une quelconque des Revendications 1 à 8, à la préparation de médicaments.

## Claims
### Claims for the following Contracting States : BE, CH, DE, GB, IT, LI, LU

1. Galenic form based on fresh plant(s), characterized in that it is in the form of a paste and is derived from a cryocomminuted form of the said plant, obtained by harsh freezing the freshly picked plant at a temperature between -80°C and -120°C, followed by at least one cryogenic comminution of the said frozen plant and then by mixing 5 to 75% by weight of the said cryocomminuted form of plant with 1 to 5% by weight of a gelling agent in a mixer under inert gas at a temperature between -5°C and -30°C and by bringing back to ambient temperature, to thus be stabilized.

2. Galenic form according to Claim 1, characterized in that the gelling agent is taken from the group containing galactomannans, natural flours, cellulose and its derivatives, crosslinked polyacrylic acids, silicas, gelatin, agar-agar, gum tragacanth or gum arabic, alginates, pectins, palmitostearates, oleates, laurates and myristates.

3. Galenic form according to Claim 1, characterized in that the fresh plant which is frozen then cryocomminuted is micronized before being incorporated into the paste form.

4. Galenic form according to any one of Claims 1 to 3, characterized in that it also contains preservatives (0.15 to 0.5% by weight).

5. Galenic form according to any one of Claims 1 to 4, characterized in that it contains in the case of internal use 0.1 to 10% by weight of a sweetener and flavourings (0.13 to 2% by weight) and in the case of external use 5 to 30% by weight of glycerol.

6. Galenic form according to any one of Claims 1 to 5, characterized in that the cryocomminuted form of plant has a particle size in the order of 100 $\mu$m.

7. Use of the galenic form according to any one of Claims 1 to 6 for external application.

8. Use of the galenic form according to any one of Claims 1 to 6 for internal application.

9. Use of the galenic form according to any one of Claims 1 to 6 in pharmacy.

10. Use of the galenic form according to any one of Claims 1 to 6 in cosmetology.

11. Use of the galenic form according to any one of Claims 1 to 6 in dietetics.

12. Process for preparing the galenic form according to any one of Claims 1 to 6, characterized in that:
   - the freshly picked plant is rendered fragile by harsh freezing at a temperature between -80°C and -120°C;
   - the said frozen plant is subject to at least one cryogenic comminution until a particle size in the order of 100 $\mu$m is obtained;
   - in that 5 to 75% by weight of cryocomminuted form of plants are mixed with 1 to 5% by weight of a gelling agent in a mixer under inert gas at a temperature between -5°C and -30°C; and

- in that the said cryocomminuted form is brought back to ambient temperature, to stabilize it.

13. Process according to Claim 12, characterized in that the plant to be processed - particularly in the case of roots - is pre-cut before cryofreezing such that the latter is rapid even at the heart of the material.

**Claims for the following Contracting State : ES**

1. Process for preparing a galenic form based on fresh plants in paste form, containing plants which have been freshly picked, frozen and then cryocomminuted, characterized in that the freshly picked plant is rendered fragile by harsh freezing at a temperature between -80°C and -120°C, is then subjected to cryogenic comminution, in that the cryocomminuted form is incorporated with the gelling agent in a mixer under inert gas at a temperature between -5°C and -30°C, and in that it is then stabilized on being brought back to ambient temperature.

2. Process according to Claim 1, characterized in that the plant to be processed - particularly in the case of roots - is pre-cut before cryofreezing such that the latter is rapid even at the heart of the material.

3. Process according to any one of Claims 1 and 2, characterized in that processing in the comminution mill may be carried out one or more times, in order to obtain a particle size in the order of 100 μm.

4. Process according to any one of Claims 1 to 3, characterized in that the paste form of fresh plants contains from 5 to 75% by weight of plants and from 1 to 5% by weight of a gelling agent (which may be natural or synthetic).

5. Process according to any one of Claims 1 to 4, characterized in that the gelling agent used is taken from the group containing galactomannans, natural flours, cellulose and its derivatives, crosslinked polyacrylic acids, silicas, gelatin, agar-agar, gum tragacanth or gum arabic, alginates, pectins, palmitostearates, oleates, laurates and myristates.

6. Process according to any one of Claims 1 to 5, characterized in that before its incorporation into the paste form the fresh plant which is frozen then cryocomminuted is previously micronized.

7. Process according to any one of Claims 1 to 6, characterized in that the paste form containing the plants also contains preservatives (0.15 to 0.5% by weight).

8. Process according to any one of Claims 1 to 7, characterized in that the paste form containing the plants contains, in the case of internal use, 0.1 to 10% by weight of a sweetener and flavourings (0.13 to 2% by weight) and in the case of external use 5 to 30% by weight of glycerol.

9. Application of paste forms containing plants according to any one of Claims 1 to 8, for preparing medicaments.

**Patentansprüche**
**Patentansprüche für folgende Vertragsstaaten : BE, CH, DE, GB, IT, LI, LU**

1. Galenische Form auf der Basis einer frischen Pflanze bzw. frischer Pflanzen, dadurch **gekennzeichnet,** daß sie pastenartige Form aufweist und sich von einem Tieftemperatur-Zerkleinerungsgut der Pflanze, erhalten durch sehr starkes Tiefgefrieren der frisch geernteten Pflanze auf eine Temperatur zwischen -80°C und -120°C, gefolgt von mindestens einer kryogenen Zerkleinerung der tiefgefrorenen Pflanze, anschließend von einem Gemisch aus 5 bis 75 Gew.-% des Tieftemperatur-Zerkleinerungsgutes der Pflanze mit 1 bis 5 Gew.-% eines Geliermittels in einem Mischer unter Inertgas bei einer Temperatur zwischen-5°C und -30°C und von einer Rückkehr auf die Raumtemperatur, um so stabilisiert zu werden, ableitet.

2. Galenische Form nach Anspruch 1, dadurch **gekennzeichnet,** daß das Geliermittel ausgewählt ist aus der Gruppe, enthaltend die Galactomannane, natürliche Mehle, Cellulose und ihre Derivate, vernetzte Polyacrylsäuren, Kieselsäuren, Gelatine, Agar-Agar, Traganthgummi oder Gummi Arabicum, Alginate, Pectine, Palmitostearate, Oleate, Laurate und Myristate.

3. Galenische Form nach Anspruch 1, dadurch **gekennzeichnet,** daß die frische, tiefgefrorene und dann kryogen zerkleinerte Pflanze vor ihrer Einarbeitung in die pastenartige Form mikrofein vermahlen worden ist.

4. Galenische Form nach einem der Ansprüche 1 bis 3, dadurch **gekennzeichnet,** das sie zusätzlich 0,15 bis 0,5 Gew.-% Konservierungsmittel enthält.

5. Galenische Form nach einem der Ansprüche 1 bis 4, dadurch **gekennzeichnet,** daß sie im Falle einer inneren Anwendung 0,1 bis 10 Gew.-% eines Süßungsmittels und 0,13 bis 2 Gew.-% Aromastoffe sowie im Falle einer äußerlichen Anwendung 5 bis 30 Gew.-% Glycerin enthält.

6. Galenische Form nach einem der Ansprüche 1 bis 5, dadurch **gekennzeichnet,** daß das Tieftemperatur-Zerkleinerungsgut der Pflanze eine Korngrößenverteilung der Teilchen in der Größenordnung von 100 $\mu$m besitzt.

7. Verwendung der galenischen Form nach einem der Ansprüche 1 bis 6 zur äußeren Anwendung.

8. Verwendung der galenischen Form nach einem der Ansprüche 1 bis 6 zur inneren Anwendung.

9. Verwendung der galenischen Form nach einem der Ansprüche 1 bis 6 in der Pharmazie.

10. Verwendung der galenischen Form nach einem der Ansprüche 1 bis 6 in der Kosmetologie.

11. Verwendung der galenischen Form nach einem der Ansprüche 1 bis 6 in der Diätetik.

12. Verfahren zur Herstellung der galenischen Form nach einem der Ansprüche 1 bis 6, dadurch **gekennzeichnet,** daß
    - die frisch geerntete Pflanze durch starkes Tiefgefrieren auf eine Temperatur zwischen -80 ° C und -120 ° C spröde gemacht wird;
    - die tiefgefrorene Pflanze mindestens einer kryogenen Zerkleinerung unterworfen wird, bis eine Korngrößenverteilung der Teilchen in der Größenordnung von 100 $\mu$m erreicht wird;
    - man 5 bis 75 Gew.-% des Tieftemperatur-Zerkleinerungsgutes der Pflanzen mit 1 bis 5 Gew.-% eines Geliermittels in einem Mischer unter Inertgas bei einer Temperatur zwischen -5 ° c und -30 ° C mischt; und
    - man das Tieftemperatur-Zerkleinerungsgut auf Raumtemperatur zurückbringt, um es zu stabilisieren.

13. Verfahren nach Anspruch 12, dadurch **gekennzeichnet,** daß die zu behandelnde Pflanze - vor allem im Falle von Wurzeln - vor dem Tiefgefrieren zerschnitten wird, damit das Tiefgefrieren, selbst im Inneren des Materials, schnell erfolgt.

**Patentansprüche für folgenden Vertragsstaat : ES**

1. Verfahren zur Herstellung einer galenischen Form auf der Basis von frischen Pflanzen in pastenartiger Form und enthaltend frisch geerntete, tiefgefrorene und schließlich kryogen zerkleinerte Pflanzen, dadurch **gekennzeichnet,** daß die frisch geerntete Pflanze durch starkes Tiefgefrieren auf eine Temperatur zwischen -80 ° C und -120 ° C spröde gemacht und dann einer kryogenen Zerkleinerung unterworfen wird und daß das kryogene Zerkleinerungsgut in einem Mischer unter einem Inertgas bei einer Temperatur von -5 ° C bis -30 ° C in das Geliermittel eingearbeitet und dadurch bei der Rückkehr zur Raumtemperatur stabilisiert wird.

2. Verfahren nach Anspruch 1, dadurch **gekennzeichnet,** daß die zu behandelnde Pflanze - vor allem im Falle von Wurzeln - vor dem Tiefgefrieren zerschnitten wird, damit das Tiefgefrieren schnell, selbst im Inneren des Materials, erfolgt.

3. Verfahren nach einem der Ansprüche 1 und 2, dadurch **gekennzeichnet,** daß ein oder mehrere Durchgänge durch die Mühle vorgenommen werden können, um eine Korngrößenverteilung der Teilchen in der Größenordnung von 100 $\mu$m zu erzielen.

4. Verfahren nach einem der Ansprüche 1 bis 3, dadurch **gekennzeichnet,** daß die pastenartige Form der frischen Pflanzen 5 bis 75 Gew.-% Pflanzen und 1 bis 5 Gew.-% eines natürlichen oder synthetischen Geliermittels enthält.

5. Verfahren nach einem der Ansprüche 1 bis 4, dadurch **gekennzeichnet,** daß das verwendete Geliermittel aus der Gruppe, enthaltend die Galactomannane, natürliche Mehle, Cellulose und ihre Derivate, vernetzte Polyacrylsäuren, Kieselsäuren, Gelatine, Agar-Agar, Traganthgummi oder Gummi Arabicum, Alginate, Pectine, Palmitostearate, Oleate, Laurate und Myristate, ausgewählt wird.

6. Verfahren nach einem der Ansprüche 1 bis 5, dadurch **gekennzeichnet,** daß die frische, tiefgefrorene und anschließend kryogen zerkleinerte Pflanze vor ihrem Einarbeiten in die pastenartige Form vorher mikrofein vermahlen wird.

7. Verfahren nach einem der Ansprüche 1 bis 6, dadurch **gekennzeichnet,** daß die pastenartige Form, die die Pflanzen enthält, ebenso 0,15 bis 0,5 Gew.-% Konservierungsstoffe enthält.

8. Verfahren nach einem der Ansprüche 1 bis 7, dadurch **gekennzeichnet,** daß die pastenartige Form, die die Pflanzen enthält, im Falle einer inneren Anwendung 0,1 bis 10 Gew.-% eines Süßungsmittels und 0,13 bis 2 Gew.-% Aromastoffe sowie im Falle einer äußerlichen Anwendung 5 bis 30 Gew.-% Glycerin enthält.

9. Verwendung der pastenartigen Formen, die die Pflanzen enthalten, nach einem der Ansprüche 1 bis 8 zur Herstellung von Medikamenten.